# EUROPEAN PATENT APPLICATION

(11) **EP 1 857 461 A1**
(43) Date of publication of application: **21.11.2007**
(21) Application number: 06728858.9
(22) Date of filing: 09.03.2006
(51) Int. Cl.: C07H 19/24, C07H 21/04, C12N 15/09

(54) **LINKER COMPOUND, PROBE, AND CARRIER HAVING PROBE IMMOBILIZED THEREON**

(30) Priority: 09.03.2005 JP 2005065948
(71) Applicant: Kyoto University, Kyoto-shi, Kyoto 606-8501 (JP); Shinwa Chemical Industries, Ltd., Kyoto-Shi, Kyoto-fu 612-8307 (JP); NGK INSULATORS, LTD., Nagoya-city, Aichi 467-8530 (JP)
(72) Inventor: MAKINO, Keisuke, ity, Gokasho, Uji-shi, Kyoto, 6110011 (JP); KODAKI, Tsutomu, , Gokasho, Uji-shi, Kyoto, 6110011 (JP); WATANABE, Seiya, ity, Gokasho, Uji-shi, Kyoto, 6110011 (JP); PACK, Seung, Pil, ity, Gokasho, Uji-shi, Kyoto, 6110011 (JP); NONOGAWA, Mitsuru, , Gokasho, Uji-shi, Kyoto, 6110011 (JP); WADA, Hiroo, tsu-cho, Fushimi-ku, Kyoto-city, Kyoto, (JP); YOSHIDA, Yasuko, u, Nagoya-city, Aichi, 4678530 (JP); YAMADA, Kazunari, u, Nagoya-city, Aichi, 4678530 (JP)
(74) Representative: Paget, Hugh Charles Edward
(86) International application number: PCT/JP2006/304641
(87) International publication number: WO 2006/095830

(57) **Abstract**

The present invention aims to provide a technique for stably immobilizing a probe on a carrier The present invention provides a linker compound represented by formula (1): 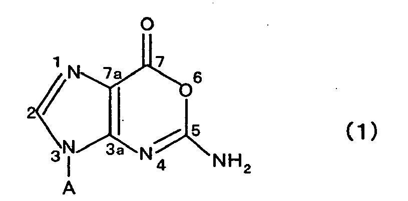 wherein A represents a hydrogen atom, another substituent, a sugar, or a derivative thereof.

SEQUENCE LISTING

<110> Kyoto University
      Shinwa Kakou Kabushiki Kaisha
      NGK Insulators, Ltd.

<120> Linker for Hybridazation probe and Use Thereof

<130> HP/FP6493233

<140> EP06728858.9
<141> 2006-03-09

<160> 6

<170> PatentIn version 3.1

<210> 1
<211> 25
<212> DNA
<213> Artificial

<220>
<223> oligonucletide probe

<400> 1
agagaaaacc ccttcaccaa agcgt                                          25


<210> 2
<211> 25
<212> DNA
<213> Artificial

<220>
<223> oligonucleotide probe

<400> 2
aagaagctga acgcttagtg aggcg                                          25


<210> 3
<211> 25
<212> DNA
<213> Artificial

<220>
<223> oligonucletide probe

<400> 3
gcaggcagat aaatcagagt ggggg                                          25


<210> 4
<211> 25
<212> DNA

<213>  Artificial

<220>
<223>  target sample

<400>  4
acgctttggt gaaggggttt tctct                                          25


<210>  5
<211>  25
<212>  DNA
<213>  Artificial

<220>
<223>  target sample

<400>  5
cgcctcacta agcgttcagc ttctt                                          25


<210>  6
<211>  25
<212>  DNA
<213>  Artificial

<220>
<223>  target sample

<400>  6
cccccactct gatttatctg cctgc                                          25

## Description

### Technical Field

The present invention relates to a linker compound suitable for use in a probe for a hybridization reaction of nucleic acids, a probe, a probe-imoobilized carrier, a method of producing the probe-immobi-lized carrier, and a method of hybridizing nucleic acids.

### Background Art

In hybridization assays in which a nucleic acid such as an oligonucleotide is used as a probe, e.g., DNA chips and DNA microarrays, it is necessary to immobilize the probe on a carrier such as a substrate or particles. For example, long-chain DNA molecules such as polymerase chain reaction (PCR) products are immobilized on a glass slide coated with polycations by means of electrostatic bonds. As regards synthesized oligonucleotides, a functional group such as an amino group or an aldehyde group is introduced at the terminal thereof in advance, and the oligonucleotides are immobilized on a glass slide having an amino group or an aldehyde group thereon by means of covalent bonds (Protein, nucleic acid, and enzyme (PNE), Vol. 43, No. 13, p. 2005).

Furthermore, oxanine is known as a damaged base from guanine that is produced when a DNA molecule comes into contact with NO or the like in living organisms (J. Am. Chem. Soc. 1996, 118, pp. 2515-2516). It is known that a DNA molecule containing oxanine as a base at a specific position in the double strand produces a cross-linked product with a DNA glycosylase. In addition, a reaction product of deoxyoxanosine with glycine, which is a deoxyribonucleotide containing an oxanine base is also known (Chem. Res. Toxicol. 2000, 13, pp. 227-230). Furthermore, it is also known that cross-linking occurs at a side chain of a specific amino-acid residue of a DNA glycosylase (J. Biol. Chem. Vol. 278, No. 27, pp. 26264-25272, 2003). It is also known that oligonucleotides containing oxanine as a base can be synthesized using PCR (Biochem., 1997, 36, pp. 8013-8019, and Biochem., 1998, 37, pp. 11592-11598).

### Disclosure of Invention

However, in all known methods of immobilizing a probe, it is difficult to stably immobilize the probe through the hybridization process, the subsequent processes, and the storage process of the immobilized carrier. In addition, when nucleic acids are introduced on a substrate or the like by means of covalent bonds, the reactivity and the specificity are not necessarily sufficient, and it is also difficult to control the immobilized form of the nucleic acids on the substrate.

Accordingly, it is an object of the present invention to provide a technique for stably immobilizing a probe on a carrier. It is another object of the present invention to provide a technique for controlling the immobilized form of the probe.

The present inventors have studied the above-described problems and focused on an irreversible reaction between oxanine, which is a base produced when a DNA molecule is damaged by NO or the like in living organisms, and an amino group. As a result, the present inventors have found that, when this base is provided in a probe and the irreversible reaction between oxanine and an amino group and the binding form thereof are utilized, the probe can be stably immobilized on a carrier. This finding led to the realization of the present invention. More specifically, the present invention provides the following means.

The present invention provides a linker compound represented by formula (1): 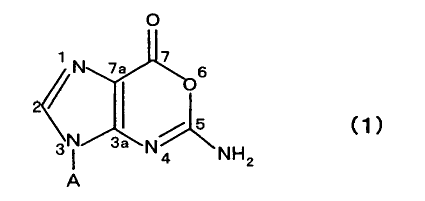 wherein A represents a hydrogen atom, another substituent, a sugar, or a derivative thereof.

In the linker compound of the invention, the linker compound is preferably a nucleoside derivative in which A is selected from ribose, deoxyribose, and a derivative thereof. It is preferable that a phosphoramidite group is introduced to the hydroxyl group at the C3' position of the ribose or the deoxyribose, and a dimethoxytrityl group is introduced to the hydroxyl group at the C5' position thereof. The linker compound may be a nucleotide derivative.

The present invention also provides a probe comprising at least one linker unit represented by formula (2): 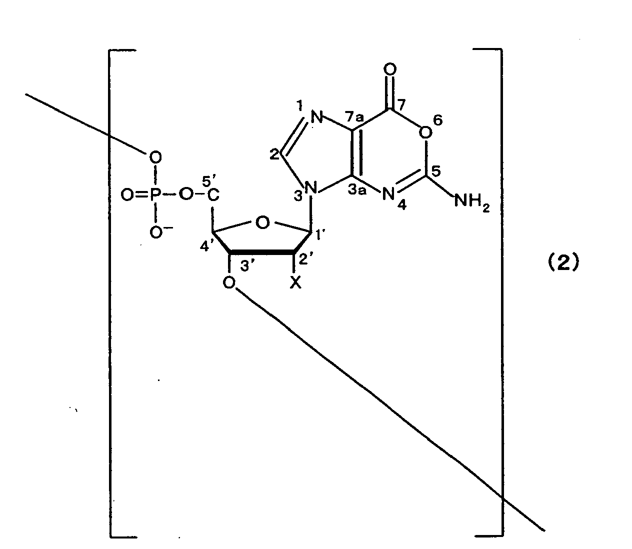 wherein X represents a hydrogen atom, a hydroxyl group, or a substituent.

The probe of the invention may be an oligonucleotide probe or a polynucleotide probe. In the probe of the invention, the linker unit may be provided at a terminal of the probe, near a terminal of the probe, or near the center of the probe. The probe may comprise a hybridization sequence complementary to a target nucleic acid. The probe may comprise the at least one linker unit, a hybridization sequence, and a spacer interposed between the hybridization sequence and the linker unit. The probe may comprise a sequence that can form a hairpin structure or a loop structure.

The present invention also provides a probe-immobilized carrier on which a probe is immobilized, where the probe is the probe of the invention in any form described above. In the probe-immobilized carrier of the invention, the probe may be immobilized by means of a covalent bond via an amino group provided on the surface of the carrier. The amino group may be provided on the carrier via a siloxane bond. In the probe-immobilized carrier of the invention, the carrier may have a form of a flat plate, a particle, or an inner layer of a capillary. The carrier may have a flat plate shape and the probe may be supplied on the carrier by spotting.

The present invention also provides a method of producing a probe-immobilized carrier comprising the steps of: preparing a liquid containing the probe of the invention in any form described above; and immobilizing the probe on a carrier having an amino group by supplying the liquid to the surface of the carrier.

The present invention further provides a method of hybridizing nucleic acids comprising the steps of: preparing the probe-immobilized carrier of the invention in any form described above; and hybridizing a probe with a target nucleic acid by supplying the target nucleic acid on the probe-immobilized carrier.

### Brief Description of the Drawings

Fig. 1 is a schematic view showing the binding form of an oxanosine-modified oligo-DNA molecule and an amino group on the surface of a carrier.
Fig. 2 is a drawing showing an example of the synthesis scheme of an oxanosine-modified oligo-DNA molecule.
Fig. 3 is a graph showing the relationship between the storage period of various substrates and the intensities of a fluorescence signal.

### Best Mode for Carrying Out the Invention

A linker compound of the present invention is a compound containing a base represented by formula (1): 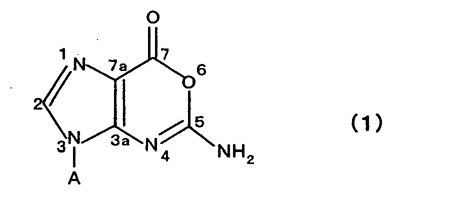 wherein A represents a hydrogen atom, another substituent, a sugar, or a derivative thereof.

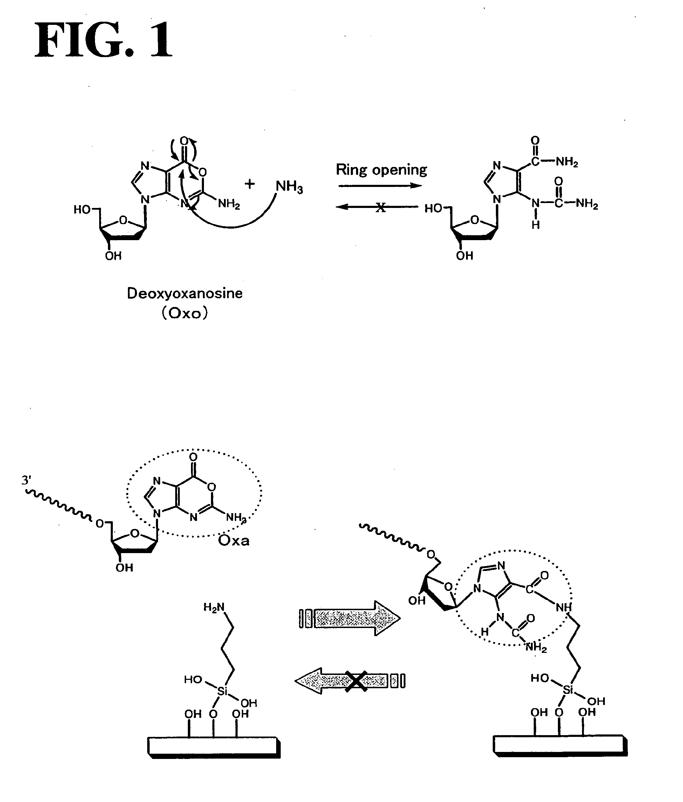

It is believed that this linker compound is immobilized on a carrier by the reaction and the binding form shown in Fig. 1. More specifically, it is believed that a carboxyl group activated by the carbodiimide in the six-membered ring moiety of the linker compound is reacted with an amino group to form a new amide bond. Since this reaction is not an equilibrium reaction but an irreversible reaction, the new amide bond is stably maintained. Accordingly, when this linker compound is linked to a probe used for a hybridization assay with a complementary nucleic acid, the probe can be bound to a carrier having an amino group or other compounds.

Since a probe containing such a linker compound as a unit is stably held on a carrier, the signal intensity obtained by hybridization and the hybridization efficiency can be stably ensured for a long time. Furthermore, since the linker compound easily reacts with an amino group on the carrier, the probe can be immobilized on the carrier with a high density. Therefore, high signal sensitivity can be obtained. Consequently, this probe can enable performance of a hybridization assay with excellent detection sensitivity and detection accuracy.

Furthermore, for such a probe, the immobilized state on a. carrier can also be stably maintained in a pretreatment process of the carrier surface, a hybridizing process, a cleaning process, and the like, which are performed in conjunction with the hybridization assay. Accordingly, regardless of such procedures, results with high repeatability can be easily obtained.

Such a probe binds to the surface of a carrier having an amino group at the linking positions of the linker compound. In addition, since a nucleotide that is another unit of the probe does not have this binding reactivity in the linker compound, other functional groups of the probe need not be protected.

Furthermore, since the linker compound of the present invention can be in the form of a nucleotide and is stable in this form, the linker compound can be easily introduced into a desired position in the probe. Accordingly, a probe can be easily prepared by using the above-described linker compound. Furthermore, the binding form of the probe to the carrier can be freely designed, and thus an immobilized form for easily obtaining hybridization efficiency can be imparted to the probe.

Furthermore, such a probe can easily form a stable bond with an amino-group-holding carrier. Therefore, such a probe can be effectively used for a carrier having a form of not only a flat plate, but also a particle, an inner layer of a capillary, or the like.

On the basis of the above reasons, a carrier on which a probe of the present invention is immobilized is suitable for a hybridization assay with a target nucleic acid.

Furthermore, on the basis of the above reasons, a hybridization method using a probe-immobilized carrier of the present invention can realize a hybridization assay with satisfactory detection sensitivity and detection accuracy.

Furthermore, on the basis of the above reasons, according a method of producing a probe-immobilized carrier of the present invention, a probe-imnobilized carrier that can enable performance of a hybridization assay with excellent detection sensitivity and detection accuracy can be produced.

A linker compound, a probe, a probe-immobilized carrier, and a method of producing a probe-immobilized carrier of the present invention will now be described. In addition, a hybridization assay using the probe-immobilized carrier will now be described.

### Linker compound

A linker compound of the present invention is represented by formula (1). In the formula, A may be a hydrogen atom, another substituent, a sugar, or a derivative thereof. When A is hydrogen, the compound represented by formula (1) is an oxanine base. This base is one of damaged bases produced by oxidative deamination of guanosine induced by NO or NO₂. For the purpose of this description, a form in which oxanine is combined with ribose (furanose type) is referred to as "oxanosine", a form in which oxanine is combined with deoxyribose (furanose type) is referred to as "deoxyoxanosine", and a form in which oxanine is combined with ribose or deoxyribose is generically referred to as "oxanine nucleoside".

Oxanine can be obtained as deoxyoxanosine by, for example, allowing deoxyguanosine to react with NO and/or NaNO₂. For example, oxanine can be obtained by allowing deoxyguanosine to react with NaNO₂ under an acidic condition, for example, using a sodium acetate buffer solution (3.0 M, pH 3.7) at 37°C for four hours. After the reaction, the reaction solution is neutralized with NaOH. The solution is appropriately distilled off under reduced pressure as required. The product is then purified by reverse-phase chromatography or the like, and dried. Thus, deoxyoxanosine can be obtained as a white powder. When a mixture (e.g., acetonitrile 10%) of a neutral buffer solution such as a sodium phosphate buffer solution (400 µM, pH 7.4) and an organic solvent such as acetonitrile is used as a mobile phase, the deoxyoxanosine can be satisfactorily separated. Oxanine can be obtained by performing acid hydrolysis of deoxyoxanosine, and then performing reverse-phase chromatography or the like on the resulting reaction solution. The acid hydrolysis can be performed under a reaction condition, for example, at 37° C for two hours using an acid such as 1 M hydrochloric acid.

In formula (1), A may be a sugar such as a pentose, e.g., ribose or deoxyribose. Examples of derivatives of a sugar include derivatives of a sugar in which a hydrogen atom or a hydroxyl group bonded to a carbon atom of the sugar is substituted with a functional group such as alkoxy, alkoxyalkyloxy, or halogen.

The linker compound of the present invention may contain oxanine in any form as long as reactivity with an amino group is ensured in the carboxyl group activated by carbodiimide. Preferably, the linker compound contains oxanine in a form in which oxanine is combined with a pentose such as ribose or deoxyribose. Regarding the bonding position of the base and the pentose, the carbon atom at the C1' position of the pentose is preferably bonded to the nitrogen (N) atom at the 3rd position of the base. When the pentose is 1-β-ribofuranosylribose or ribofuranosyldeoxyribose, the linker compound has a form of a nucleoside derivative. As described above, such a linker compound having a form of a nucleoside derivative can be easily obtained from deoxyguanine or deoxyguanosine.

Various modifications suitable for obtaining an oligonucleotide by a chemical synthesis may be performed on such a linker compound having a form of a nucleoside derivative. For example, the hydroxyl group at the C3' position and the hydroxyl group at the C5' position of ribose or deoxyribose are modified as needed. Preferably, a phosphoramidite group is introduced to the hydroxyl group at the 3' position, and a dimethoxytrityl group is introduced to the hydroxyl group at the 5' position. Such a modifying group can be introduced by appropriately employing a known method of chemically synthesizing a nucleic acid. An example of a linker compound of the present invention suitable for a chemical synthesis of a nucleic acid by a phosphoramidite method is shown below. 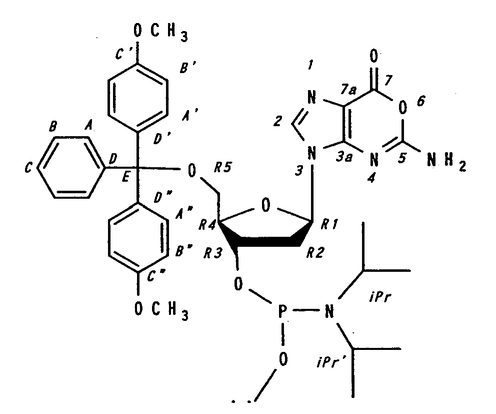 In the formula, R1 represents 2-cyanoethyl, 4-nitrophenylethyl, 2-trimethylsilylethyl, or methyl.

Such a linker compound of the present invention can be obtained by employing a known method of synthesizing a monomer for a nucleic acid chemical synthesis. For example, dimethoxytrityl chloride is added to a suspension (containing a solvent such as dimethylformamide (DMF)) of deoxyoxanosine, and imidazole and diisopropylethylammonium mesylate are then added to the mixture. The resulting reaction solution is homogenized by stirring. The reaction solution is then poured into a large amount of water, thus allowing a precipitate to be obtained. The precipitate is dried to obtain a crude product. This crude product is re-dissolved in an appropriate solvent, such as dichloromethane, and is then recrystallized in hexane. Thus, DMT-deoxyoxanosine can be obtained.

Furthermore, a phosphoramidite group can be introduced to DMT-deoxyoxanosine as follows. First, DMT-deoxyoxanosine is dissolved in an appropriate solvent, 2-cyanoethyl-N,N,N',N'-diisopropylphosphoramide is then added to the solution. After a reaction is sufficiently performed, the reaction is terminated by adding methanol, diisopropylethylamine, and ethyl acetate. The reaction solution is washed with a 10% sodium hydrogencarbonate solution, and the organic phase is then dehydrated and concentrated. The concentrate is re-dissolved in an appropriate solvent such as dichloromethane and separated by normal-ghase chromatography. For example, a mixture of ethyl acetate, dichloromethane, and methanol (ethyl acetate:dichloromethane:methanol = 65:35:0.33) can be used as the mobile phase.

Furthermore, the linker compound of the present invention preferably has a form of a phosphate ester at the hydroxyl group bonded to the carbon atom at the 5th position of ribose or deoxyribose. The phosphate ester is preferably a triphosphate. In this case, the linker compound can have a form of a nucleotide derivative. Such a probe compound can be preferably used for obtaining a nucleic acid such as a long-chain DNA molecule having 100 bases or more by a gene amplifying method such as a PCR method. An example of a linker compound of the present invention used as a nucleotide derivative is shown by a formula below.

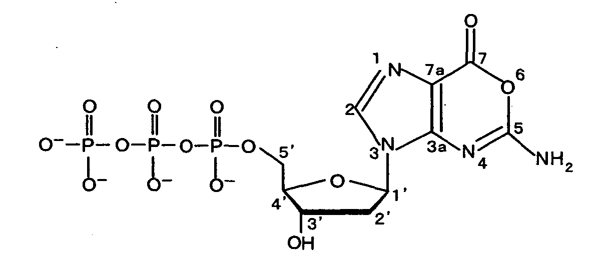

### Probe

A probe of the present invention includes such a linker compound as a linker unit. The probe is used for a hybridization assay of a nucleic acid, i.e., an oligonucleotide or a polynucleotide. Accordingly, the probe can have a form of a polynucleotide or an oligonucleotide such as DNA, RNA, or cDNA. Alternatively, the probe may be PNA. Herein, the term "oligonucleotide" mainly means a polymer composed of several nucleotides to one hundred and several tens of nucleotides or less, more preferably, about one hundred nucleotides or less and mainly includes a polymer obtained by a chemical synthesis or the like. The term "polynucleotide" mainly means a polymer having a chain longer than the chains of oligonucleotides, and includes PCR products and the like.

The probe of the present invention is preferably an oligodeoxyribonucleotide or a polydeoxyribonucleotide containing deoxyribose. In the probe of the present invention, the linker unit is preferably included as the same type of nucleotide derivative as another monomer unit included in the probe.

An oligonucleotide including a linker unit as a nucleotide derivative can be prepared by employing a known method of preparing a probe. When a DNA chain elongation method using a DNA polymerase is employed, for example, deoxyoxanosine triphosphate (dOTP) is prepared as the linker compound. It is believed that oxanine forms two hydrogen bonds with cytosine or thymine (preferably, cytosine) to form a stable base pairing. In consideration of such a base pairing, a desired template and a primer ranging from the 5' terminal to a desired position to which the linker compound is introduced are prepared. A first elongation step is performed in a reaction system containing only dOTP. Subsequently, the remaining dOTP is removed, and second and the subsequent elongation steps are normally performed in the presence of required dNTP (wherein N is at least one of A, T, C, and G). Subsequently, as required, when an elongation step is performed using only dOTP, the linker compound can be introduced to at least two desired sites.

When a phosphoramidite method is employed, for example, a deoxyoxanosine derivative modified with a phosphoramidite group described above is prepared. The derivative is added to an oligodeoxynucleotide under synthesizing in a desired introduction order, and a chain elongation reaction is performed. The linker compound can also be introduced to one or two or more desired sites of the probe by this method.

The probe of the present invention functions in the form of a single strand nucleic acid in hybridization. However, the probe may have a form of a double strand in the acquisition, the synthesis, or the immobilization. When the probe has a form of a single strand, the probe may have a loop or hairpin site.

The probe can include one, or two or more linker units. The linker unit can be provided at the 5' terminal, which is exclusively selected as a carrier immobilization site in a probe. Alternatively, the linker unit may be provided at other sites such as the 3' terminal. For example, the linker unit may be provided near the center of the probe. Alternatively, the probe may be designed so that the linker unit is provided at the 5' terminal side or the 3' terminal side, and when the probe is immobilized on a carrier, a hairpin structure or a loop structure is partly formed at the upstream side and the downstream side of the probe including the linker compound site. In particular, when the linker unit has a form of a nucleotide derivative, as described above, the linker unit can be introduced to any position by a chemical synthesis or an enzymatic chain elongation method.

In the probe, preferably, a region hybridizing with a target nucleic acid does not include the linker unit. Accordingly, when the linker unit is introduced near the center of the probe or near a terminal of the probe, a spacer region that is not involved with hybridization is preferably formed near the linker unit.

When the linker unit is introduced to, for example, the 5' terminal of the probe, such a spacer region is similarly formed. In this case, from the 5' terminal side, the linker unit, the spacer region, and the hybridizing region are disposed in that order.

### Probe-immobilized carrier

A probe-immobilized carrier is a solid-phase carrier on which the probe of the present invention is immobilized. The solid-phase carrier is not particularly limited, and various known types of carriers used for a hybridization assay can be used. Examples of the solid-phase carrier include glass; ceramics; polymers such as polyethylene terephthalate, cellulose acetate, polycarbonate, polystyrene, and poly (methyl methacrylate); porous glass; porous ceramics; porous silicon, short fibers; and membrane filters. Among these, glass, silicon, polymers are preferred.

The form of the carrier is also not particularly limited as long as the carrier can immobilize the probe and has a surface (including an inner surface) on which a target nucleic acid can be supplied. Examples of the form of carrier include a flat plate such as a substrate, a particle such as a bead, a fiber, and a capillary in which an inner surface thereof is used as the immobilizing surface.

The carrier of the present invention is provided with an amino group (-NR¹R²) on the immobilizing surface in order to immobilize the probe. R¹ and R² are each independently a hydrogen atom or a substituent. The substituent is a linear alkyl group having 1 to 18, preferably 1 to 6 carbon atoms. In view of the reactivity with the linker compound, the amino group is preferably a primary amino group.

The carrier including an amino group can include at least one amino-acid residue composed of, for example, lysine, histidine, or arginine. Such an amino-acid residue may constitute the carrier surface as a coating layer in the form of a polymer. Alternatively, a carrier provided with an amino group via a siloxane bond can be obtained by introducing a mono-, di-, or tri-alkoxysilane having an amino-group-containing group into a silanol group of a carrier including the silanol group, such as a glass carrier. Preferably, the carrier is designed so that the amino group is provided at a terminal. The amino-group-containing group can include an amino group via an appropriate spacer portion. The spacer portion is preferably an alkyl group having 2 to 10 carbon atoms or a group in which some of the carbon atoms of the alkyl group is substituted with a nitrogen (N) atom or a sulfur (S) atom. The alkyl group may be linear or branched, but the alkyl group is preferably linear.

Examples of the alkoxyl group in the alkoxysilane include a methyl group, an ethyl group, a n-propyl group, an isopropyl group, a n-butyl group, a sec-butyl group, and a tert-butyl group. The remaining group (the group bonded to Si other than the alkoxyl group and the amino group-alkyl-amino group) in the alkoxysilane is preferably an alkyl group. An alkyl group having about 1 to 10 carbon atoms is preferably used as the alkyl group. Examples thereof include a methyl group, an ethyl group, a n-propyl group, an isopropyl group, a n-butyl group, a sec-butyl group, a tert-butyl group, a n-hexyl group, a cyclohexyl group, a n-octyl group, a tert-octyl group, and a n-decyl group. The alkoxysilane introduced to the silanol group is not limited thereto, and various other silane compounds can also be used. Examples of the silane compounds that can be used for the carrier are shown below.

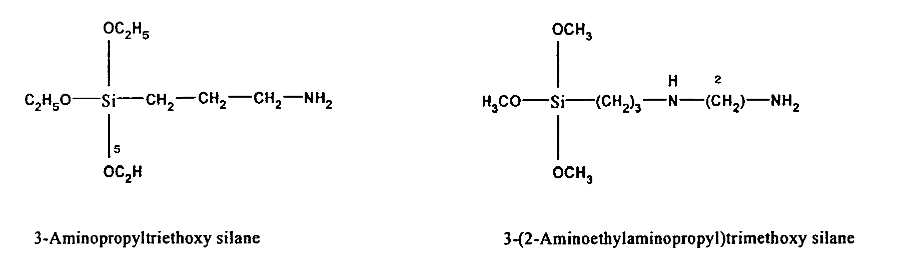

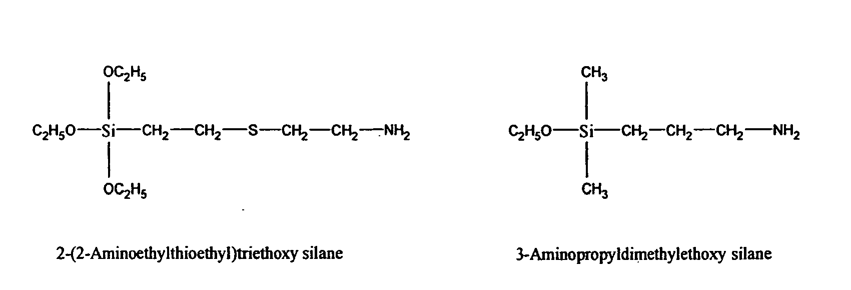

In the probe-immobilized carrier of the present invention, the probe is immobilized in a state in which a carboxyl group activated by carbodiimide in oxanine of the linker compound is reacted with an amino group on the carrier surface to form an amide bond (see Fig. 1).

The inmbj-lized form of the probe is different according to the introduction position of the linker compound in the probe. When the linker compound is provided at the 5' terminal, the probe would be immobilized in the form of a single strand. When the linker compound is provided near the center of the probe, the probe would be immobilized in the form of a U shape or the like. When the probe has, for example, a sequence that can form base pairings facing a region near the introduction site of the linker compound, a hairpin structure or a loop structure would be formed near the introduction site of the linker compound.

### Method of producing probe-immobilized carrier

A probe-immobilized carrier can be obtained by preparing a liquid containing a probe, supplying the liquid on the surface of a carrier, and allowing oxanine of the linker compound in the probe to react with an amino group provided on the carrier. Any known method of supplying a probe on a carrier can be employed. For example, in order to prepare, as a probe-immobilized carrier, an array in which a large number of probe spots are arrayed on the surface of a substrate or the like, probe-containing droplets are supplied in the form a spot using a contact type device with pins, a Bubble-Jet (registered trademark) type device, or the like. In order to supply a probe on particles such as beads, for example, the beads are dipped in a probe-containing solution, or the probe-containing solution is sprayed on the beads.

The probe can be immobilized on the carrier by a known procedure such as spotting or dipping of a probe solution. When the probe solution is supplied to the carrier including amino groups, the linker compound can immediately react with the amino groups. An organic solvent may be added to the probe solution. In addition, the probe solution may be controlled to be neutral or slightly acidic as required. The temperature and the incubation time can be appropriately determined according to the types of carrier and probe used. For example, the temperature may be determined in the range of 30° C to 90° C, and the reaction time may be in the range of several tens of minutes to one hour or about several hours. After the probe solution is supplied to the surface of the carrier, steps of rehydration, blocking, denaturation, washing, drying, and the like can be performed.

### Hybridization method

A method of hybridizing nucleic acids of the present invention includes preparing the probe-imnobilized carrier of the present invention, and supplying nucleic acids to the probe-imnobilized carrier to hybridize the probe and a target nucleic acid. In this hybridization method, various types of target nucleic acids prepared by a known method can be supplied in accordance with a common procedure to perform hybridization as far as the probe-immobilized carrier of the present invention is used.

### EXAMPLE 1

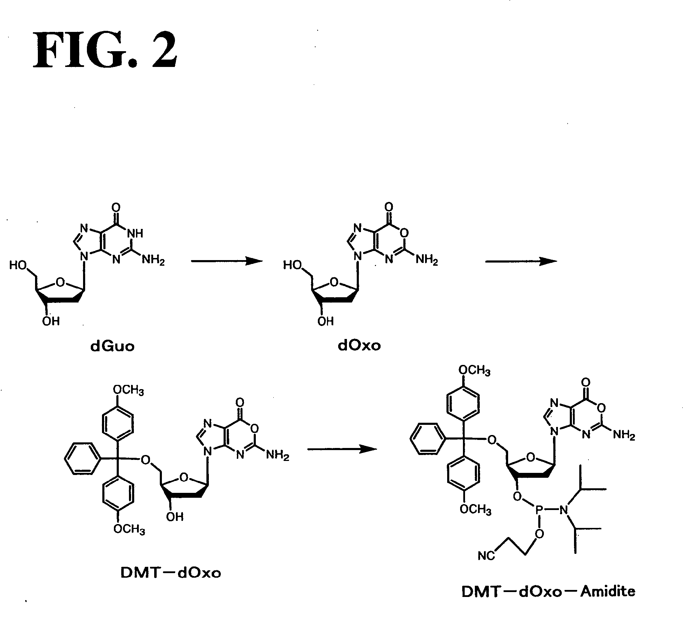

The present invention will now be specifically described using examples, but the present invention is not limited to these examples. Fig. 2 shows a synthesis scheme of a deoxyoxanosine-modified oligo-DNA molecule in Examples 1 to 4.

### Synthesis of 2'-deoxyoxanosine

First, 3 mmol of deoxyguanine was incubated in 400 mL of a sodium acetate buffer (3.0 M, pH 3.7) containing a 100 mM NaNO₂ solution at 37° C for four hours. After neutralization with a 5 M NaOH solution, half of the solvent was distilled off under reduced pressure with a rotary evaporator. The reaction solution was then filtered with a Millex-GS (0.22 µm: Millipore (Bradford, MA)) and introduced into a preparative reverse-phase HPLC system (Tosho PX-8010 (controller), CCPM (pump), and UV-8010 (UV detector) (manufactured by Tosoh Co., Tokyo, Japan), Ultron VX-ODS column (250 × 20 mm, 5 µm, manufactured by Shinwa Chemical Industries, Ltd.)). A sodium phosphate buffer (400 µM, pH 7.4) containing 10% acetonitrile was used as a mobile phase, and isocratic elution was performed at a flow rate of 6 mL/min. The fraction of deoxyoxanosine was further purified and then freeze-dried. Thus, deoxyoxanosine could be obtained in the form of a white powder (0.4 mmol. yield: 10%).

### NMR data of 2-deoxyoxanosine:

¹H NMR (600 MHz, D₂O at 30°C): δ = 7.98 (s, 1H, H-2), 6.26 (dd, 1H, H-1'), 4.61 (ddd, 1H, H-3'), 4.12 (ddd, 1H, H-4'), 3.79 (m, 2H, H-5',5"), 2.77 (ddd, 1H, H-2'), 2.52 (ddd, 1H, H-2"); UV: λₘₐₓ = 245, 286 (pH 7); MS(FAB) m/z 269 (M + H⁺)

### EXAMPLE 2

### Synthesis of 5'-O-(4,4'-dimethoxytrityl)-2'-deoxyoxanosine (DMT-dOxo)

4,4'-Dimethoxytrityl chloride (0.4 mmol) was added to a suspension of deoxyoxanosine (0.4 mmol, 3 mL of dry DMF). Imidazole (0.8 mmol) and diisopropylethylammonium mesylate (0.8 mmol) were then added to the reaction mixture.

The reaction mixture was stirred at room temperature for six hours, thereby allowing a homogenized reaction solution to be prepared. The reaction solution was poured into 900 mL of water, and the resulting precipitate was collected by filtration and dried at room temperature for 24 hours. The crude product was re-dissolved in dichloromethane and recrystallized in hexane. Thus, DMT-deoxyoxanosine was obtained in the form of a white powder (0.22 mmol, yield: 69%).

### NMR data of DMT-dQxo:

¹H NMR (600 MHz, DNSO-d₆ at 30° C) : δ = 7.88 (br, 2H, NH₂), 7.80 (s, 1H, H-2), 7.31-6.74 (m, 13H, Ar-H), 6.04 (dd, 1H, H-1'), 5.26 (s, 1H, 3'-OH), 4.39 (m, 1H, H-3'), 3.85 (m, 1H, H-4'), 3.75 (s, 6H, Ar-OCH₃), 3.06 (m, 2H, H-5',5''), 2.54 (ddd, 1H, H-2'), 2.22 (ddd, 1H, H-2''); MS(FAB) m/z 571 (M + H⁺)

### EXAMPLE 3

### Synthesis of 5'-O-(4,4'-dimethoxytrityl)-2'-deoxyoxanosine-3' -O- (2-cyanoethyl) -N.N-diisopropylphosphoramidite (DMT-dOxo-amidite)

First, 0.35 mmol of DMT-deoxyoxanosine was dissolved in 3 mL of anhydrous acetonitrile containing 0.34 mmol of tetrazole. Subsequently, 0.525 mmol of 2-cyanoethyl-N,N,N',N'-diisopropylphosphoramide was added to the solution. This homogeneous mixture was stirred at roan temperature for four hours. Fina.Ll.y, the reaction was terminated by the addition of 1 mL of methanol followed by 0.15 mL of diisopropylethylamine and 3 mL of ethyl acetate. Subsequently, the solution was washed with 3 mL of a 10% NaHCO₃ solution three times. The organic phase was dried with sodium sulfate and then concentrated, thus allowing a viscous oil to be obtained. This crude product was re-resolved in 2 mL of dichloromethane, and the solution was then introduced into preparative normal-phase HPLC system. A mixture of ethyl acetate, dichloromethane, and methanol (ethyl acetate:dich1oranethane:methanol = 65:35:0.33) was used as a mobile phase, and isocratic elution was performed at a flow rate of 6 mL/min. The fraction of the target compound was distilled off under reduced pressure : Thus, DMT-dOxo-amidite was obtained in the form of a white powder (0.29 mmol , yield 72.5%).

### NMR data:

¹H NMR (600 MHz, DMSO-d₆ at 30° C): δ = 7.88 (br, 2H, NH₂), 7.80 (s, 1H, H-2), 7.31-6.74 (m, 13H, Ar-H), 6.04 (dd, 1H, H-1'), 5.26 (s, 1H, 3'-OH), 4.39 (m, 1H, H-3'), 3.85 (m, 1H, H-4'), 3.75 (s, 6H, Ar-OCH₃), 3.06 (m, 2H, H-5',5 "), 2.54 (ddd, 1H, H-2'), 2.22 (ddd, 1H, H-2"); MS(FAB) m/z 571 (M + H⁺)

### EXAMPLE 4

### Synthesis and purification of oxanosine-modified oligo-DNA probes

The synthesis of oligodeoxynucleotide probes was started from a 5'-DMr-DNA base bound to a controlled pore glass (CPG) support (0.2 or 1 µmol scale) using a phosphoramidite method. The sequences for hybridization of the prepared probes are described below. DMT-dOxo-amidite was introduced into the 5' terminal in the final step after the synthesis of the following sequences.
Oxanosine-modified oligo-DNA probe 1: 5' -agagaaaaccccttcaccaaagcgt-3' (sequence No. 1)
Oxanosine-modified oligo-DNA probe 2: 5'-aagaagctgaacgcttagtgaggcg-3' (sequence No. 2)
Oxanosine-modified oligo-DNA probe 3: 5'-gcaggcagataaatcagagtggggg-3' (sequence No. 3)

For coupling deoxynucleoside phosphoramidites, the standard protocol was used with a DNA synthesizer (ABI). The 5'-terminal dimethoxytrityl group was not removed using the synthesizer. Each of the oligomer samples was cleaved from the CPG support and deprotected by being treated with a 0.5 M NaOH solution at 37° C for 24 hours. In the chemical synthesis of this example, the coupling rate was 95% or more in each step.

For each sample, the solution was neutralized with a 2 M TEAA buffer (pH 7.0) and then desalinated with a Poly-Pak cartridge (manufactured by Glen Researches Corporation). Furthermore, the resulting product was purified by reverse-phase HPLC with a gradient using a mobile phase of 5%(0 minutes)-5%(5 minutes)-30%(25 minutes)-40%(30 minutes)-5%(37 minutes) acetonitrile in 100 mM TEAA (pH 7.0). The flow rate was 2 mL/min.

The purified product was treated with 80% acetic acid at room temperature for 30 minutes and then dried under reduced pressure. The product was extracted with diethyl ether. The aqueous phase was subjected to the final confirmation by reverse-phase HPLC under the same gradient condition as mentioned above (except that the flow rate was 1 mL/min) . Furthermore, the purified oligo-DNA sample was treated with nuclease PI and alkaline phosphatase in 100 µL of a sodium acetate buffer (pH 4.9, containing 0.5 mM zinc chloride). The reaction solution was directly introduced into a reverse-phase HPLC system, and elution was performed with a linear gradient using a mobile phase of 0%(0 minutes)-15%(15 minutes) acetonitrile in 100 mM TEAA. The flow rate was 1 mL/min. The digested nucleotide samples thus obtained were analyzed using the resulting chromatograms and it was confirmed that deoxyoxanosine was introduced into the synthesized oligo-DNA probes.

### EXAMPLE 5

### Preparation of amino-group-containing glass substrate

Glass slides (25.4 mn × 76.2 mm × 1 mm) were washed with acetone and treated in an ultrasonic bath containing a piranha solution (concentrated sulfuric acid:30% H₂O₂ = 7:3 in volume ratio) for 30 minutes. The glass slides were then washed with a large amount of water and dried in a desiccator for one hour while suction was applied to the desiccator by means of reduced pressure.

The washed substrates were further washed with methanol for 10 minutes, a mixture of methanol and toluene (1:1) for 10 minutes, and toluene for 10 minutes. Each of the substrates was immersed in a 2% solution of a silane compound (3-aminopropyltriethoxysilane, 3-(2-aminoethylaminopropyl)trimethoxysilane, 2-(2-aminoethylthioethyl)triethoxysilane, or 3-aminopropyldimethylethoxysilane) for five hours, and stirring was performed at 25° C for five hours with a rotating stirrer (70 rpm).

After silanization, the glass slides were washed with toluene for 10 minutes, a mixture of methanol and toluene (1:1) for 10 minutes, and methanol for 10 minutes, and then dried in a hot air oven at 100° C for one hour. The glass slides were cooled to room temperature. The glass slides were then washed with toluene for 10 minutes, a mixture of methanol and toluene (1:1) for 10 minutes, and methanol for 10 minutes, and then dried in a desiccator while suction was applied to the desiccator by means of reduced pressure. Furthermore, each of these modified glass slides was then immersed in a 2% silane compound solution for eight hours and then shaken at 25° C for eight hours. The glass slides were then dried with hot air and washed in the same manner (methanol was used in the last step). The glass slides were dried with an air blow, and stored in a desiccator under reduced pressure.

### EXAMPLE 6

### Immobilization of oxanosine-modified oligo-DNA probes on substrates

Among the amino-group-modified glass slides prepared in Example 5, glass slides modified with 3-aminopropyltriethoxysilane were used as substrates (hereinafter referred to as "example substrates") serving as a carrier. As substrates 1 for a comparative example, aldehyde-modified glass slides (manufactured by TeleChem International, Inc., for amino-group-modified oligo-DNA samples, hereinafter referred to as "comparative example substrates 1") were used. As substrates 2 for another comparative example, epoxy-modified glass slides (manufactured by MMG Biotech, for amino-group-modified oligo-DNA samples, hereinafter referred to as "comparative example substrates 2") were used. Each of the oligo-DNA probes was obtained by a chemical synthesis and an amino group was introduced at the 5' terminal thereof.

As oxanosine-modified oligo-DNPr probes, three types of the oligo-DNA probes synthesized in Example 4 were used. As amino-group-modified oligo-DNA probes used for the comparative example substrates 1 and 2, oligo-DNA probes in which an amino group was introduced at the 5' terminal of each of the hybridization sequences described in sequence Nos. 1 to 3 were prepared.

Subsequently, the oligo-DNA probes for the Example, Comparative Example 1, and Comparative Example 2 were dissolved in PBS (Phosphate Buffered Saline, pH 7.6) to prepare solutions each having an oligo-DNA probe concentration of 50 pmol/µL. These solutions were used as probe solutions. These probe solutions were spotted on the example substrates, the comparative example substrates 1, and the comparative example substrates 2 with a GENESHOT (registered trademark) spotting device in an amount of about 200 pL per spot.

The following procedures were performed for the three types of substrates thus prepared.

### Example substrates

After the oxanosine-modified oligo-DNA solution was spotted, the substrates were baked at 80°C for one hour. The substrates were placed in a glass slide rack and immersed in a succinic anhydride solution (which was prepared by dissolving 2.5 g of succinic anhydride in 160 mL of 1-methyl-2-pyrrol:Ldimone, and adding 17.5 mL of a 0.2 M aqueous sodium borate solution (pH 8.0) to the solution) for 20 minutes. The whole glass slide rack was then immersed in sterile water, and the substrates were washed by gently reciprocating the glass slide rack vertically ten times. Furthermore, the whole slide rack was immersed in fresh sterile water, and the substrates were washed by gently reciprocating the glass slide rack vertically ten times. Subsequently, the glass slide rack was immersed in boiling sterile water for three minutes. The remaining moisture was removed by absorption, and the substrates were immediately immersed in cold ethanol for one minute to remove moisture. The remaining ethanol was removed by absorption, and the substrates were dried at room temperature.

### Comparative example substrates 1

After the amino-modified oligo-DNA solution was spotted, the substrates were baked at 80° C for one hour. The substrates were placed in a glass slide rack and immersed in a NaBH₄ solution (which was prepared by dissolving 1.5 g of NaBH₄ in 40 mL of ethanol and 135 mL of PBS) for five minutes. The whole glass slide rack was then immersed in sterile water, and the substrates were washed by gently reciprocating the glass slide rack vertically ten times. Furthermore, the whole slide rack was immersed in fresh sterile water, and the substrates were washed by gently reciprocating the glass slide rack vertically ten times. Subsequently, the slide rack was immersed in boiling sterile water for three minutes. The remaining moisture was removed by absorption, and the substrates were immediately immersed in cold ethanol for one minute to remove moisture . The remaining ethanol was removed by absorption, and substrates were dried at room temperature.

### Comparative example substrates 2

After the amino-modified oligo-DNA solution was spotted, the substrates were incubated at a relative humidity of 45% and 42° C for one night. The substrates were then placed in a glass slide rack. The substrates were washed in a 0.2% aqueous SDS solution by shaking the glass slide rack at room temperature for two minutes. Furthermore, the substrates were washed in sterile water by shaking the glass slide rack at room temperature for one minute, and then washed in fresh sterile water by shaking the glass slide rack at room temperature for one minute. Subsequently, the substrates were immersed in sterile water at 50° C for 20 minutes and dried by being subjected to centrifugation.

### Preparation of hybridization samples

Oligo-DNA samples which had complementary sequences (sequence Nos. 4 to 6) to the hybridization sequences of the sequence Nos. 1 to 3 of the oligo-DNA probes, respectively, and whose 5' terminals were labeled with Cy3 were prepared. Each of the oligo-DNA samples was dissolved in sterile water and used. The final concentration of each solution was 100 nM.
Complementary strand oligo-DNA sample 1: 5'-acgctttggtgaaggggttttctct-3' (sequence No. 4)
Complementary strand oligo-DNA sample 2: 5'-cgcctcactaagcgttcagcttctt-3' (sequence No. 5)
Complementary strand oligo-DNA sample 3: 5'-cccccactctgatttatctgcctgc-3' (sequence No. 6)

### Hybridization

First, 20 × SSC and 10% SDS solutions were added to each of the hybridization samples whose concentration had been adjusted so that the final concentrations were 5 × SSC and 0.5% SDS. Subsequently, 25 µL of each of these samples was dropped on each of the three types of substrates, and a cover glass was gently placed thereon. The substrates were incubated at a relative humidity of 100% and 42° C for one night. Subsequently, the substrates were shaken in a solution of 2 × SSC and 0.1% SDS at room temperature for five minutes. The substrates were then shaken in a 1 × SSC solution at room temperature for five minutes and then shaken in a 0.1 × SSC solution at room temperature for five minutes. The substrates were then dried by being subjected to centrifugation at 1,000 rpm for three minutes.

### Measurement and digitization of fluorescence signals

A measurement was performed with a ScanArray 4000 (trademark) (manufactured by Packard BioScience) while the laser power and a photomultiplier (PMT) were appropriately adjusted. The fluorescence signal of each spot was digitized using a GenePixPro (trademark) (manufactured by Axon Instruments).

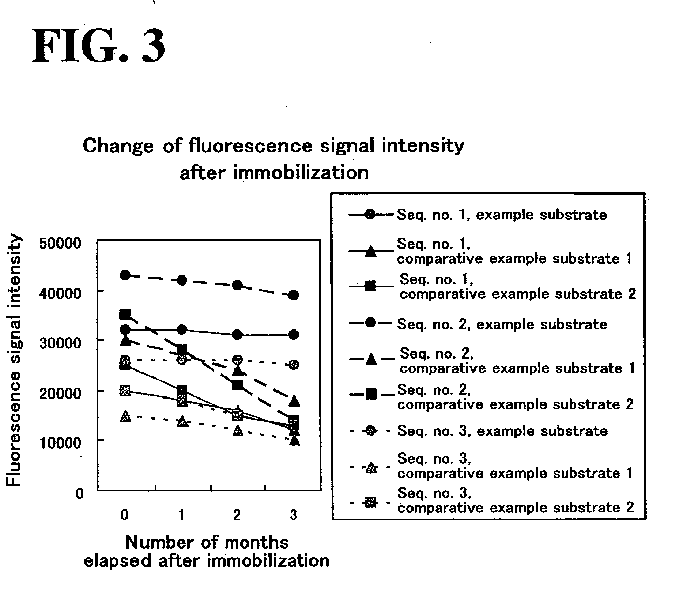

In the Example, Comparative Example 1; and Comparative Example 2, other oligo-DNA-immobilized substrates that were prepared at the same time were stored in a desiccator at room temperature. Each of the three types of substrates was taken out from the desiccator after each month, and hybridization was performed. The fluorescence signals were measured and digitized. The results are shown in Fig. 3.

As shown in Fig. 3, in the example substrate, the fluorescence signal intensities immediately after the preparation were high. Thereafter, a decrease in the fluorescence signal intensities was barely observed. In contrast, in the comparative example substrates 1 and 2, initial fluorescence signal intensities were low, and in addition, the signal intensities decreased as the storage period increased. Accordingly, these results show that, in the substrates on which the oligo-DNA probes of the present invention are immobilized, immobilization can be excellently performed, hybridization can be performed with a high hybridization efficiency, and such properties can be stably maintained even after storage.

This application claims the priority of Japanese Patent Application No. 2005-065948 filed on March 9, 2005, which is hereby incorporated by reference herein in its entirety.

### Industrial Applicability

The present invention can be used for a technical field related to hybridization of nucleic acids.

### Sequence Listing Free Text

Sequence Nos. 1 to 3: oligonucleotide probe Sequence Nos. 4 to 6: target sample

## Claims

1. A linker compound represented by formula (1): 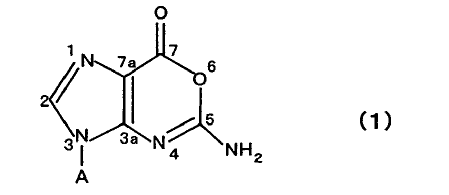 wherein A represents a hydrogen atom, another substituent, a sugar, or a derivative thereof.

2. The linker compound according to Claim 1, wherein the linker compound is a nucleoside derivative in which A is selected from ribose, deoxyribose, and a derivative thereof.

3. The linker compound according to Claim 2, wherein a phosphoramidite group is introduced to the hydroxyl group at the C3' position of the ribose or the deoxyribose, and a dimethoxytrityl group is introduced to the hydroxyl group at the C5' position thereof.

4. The linker compound according to Claim 2, wherein the linker compound is a nucleotide derivative.

5. A probe comprising:
at least one linker unit represented by formula (2): 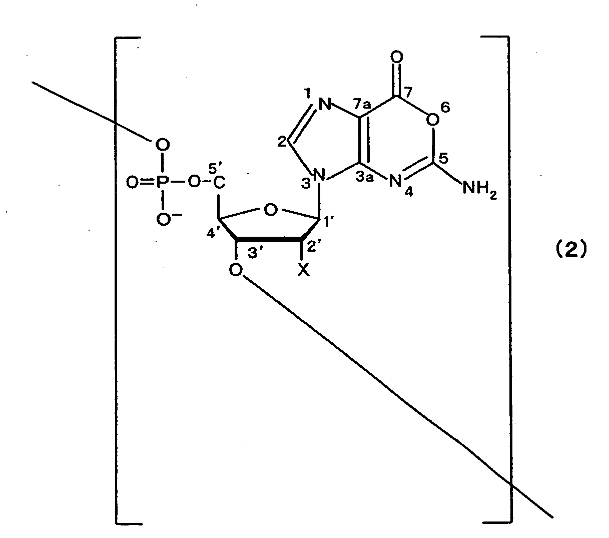
wherein X represents a hydrogen atom, a hydroxyl group, or a substituent.

6. The probe according to Claim 5, wherein the probe is an oligonucleotide probe or a polynucleotide probe.

7. The probe according to Claim 5 or 6, wherein the probe comprises a sequence that can form a hairpin structure or a loop structure.

8. The probe according to any one of Claims 5 to 7, wherein the linker unit is provided at a terminal of the probe, near a terminal of the probe, or near the center of the probe.

9. The probe according to any one of Claims 5 to 8, wherein the probe comprises the at least one linker unit, a hybridization sequence, and a spacer interposed between the hybridization sequence and the linker unit.

10. The probe according to any one of Claims 5 to 9, wherein the probe comprises a hybridization sequence complementary to a target nucleic acid.

11. A probe-immobilized carrier on which a probe is immobilized,
wherein the probe is the probe according to any one of Claims 5 to 10.

12. The probe-immobilized carrier according to Claim 11, wherein the probe is immobilized by means of a covalent bond via an amino group provided on the surface of the carrier.

13. The probe-immobilized carrier according to Claim 12, wherein the amino group is provided on the carrier via a siloxane bond.

14. The probe-immbilized carrier according to any one of Claims 11 to 13, wherein the carrier has a form of a flat plate, a particle, or an inner layer of a capillary.

15. The probe-immobilized carrier according to any one of Claims 11 to 14, wherein the carrier has a flat plate shape and the probe is supplied on the carrier by spotting.

16. A method of producing a probe-immobilized carrier comprising the steps of:
preparing a liquid containing the probe according to any one of Claims 5 to 10; and
immobilizing the probe on a carrier having an amino group by supplying the liquid to the surface of the carrier.

17. A method of hybridizing nucleic acids comprising the steps of:
preparing the probe-immobilized carrier according to any one of Claims 11 to 15; and
hybridizing a probe with a target nucleic acid by supplying the target nucleic acid on the probe-immobilized carrier.
